# EUROPEAN PATENT APPLICATION

(11) **EP 4 218 772 A1**
(43) Date of publication of application: **02.08.2023**
(21) Application number: 22153401.9
(22) Date of filing: 26.01.2022
(51) Int. Cl.: A61K 33/00, A61P 11/00

(54) **LITHIUM SALT FOR USE IN TREATMENT OF PULMONARY HYPERTENSION**

(71) Applicant: Charité - Universitätsmedizin Berlin, 10117 Berlin (DE)
(72) Inventor: Kübler, Wolfgang, 10965 Berlin (DE); Solymosi, Philip, 16727 Oberkrämer (DE)
(74) Representative: Gulde & Partner

(57) **Abstract**

The present invention is directed to lithium salt or a pharmaceutical composition comprising the same for use in treatment or prevention of pulmonary hypertension.

## Description

### BACKGROUND OF THE INVENTION:

About 1% of the adult population suffers from pulmonary hypertension (PH).

The characteristic feature of pulmonary hypertension (PH) is increased blood pressure in the pulmonary arteries. This is basically an increase in the pulmonary arterial mean pressure (mPAP) to greater than 25mmHg at rest. Morbidity and mortality of affected patients increases with the degree of PH. In the long term, PH can lead to strain on the heart muscle, causing the right ventricle in particular to dilate and lose strength, so that it can ultimately no longer transport the necessary amount of blood. Typical signs of pulmonary hypertension are narrowed pulmonary arteries with a thickened vascular wall. As a direct consequence, the oxygen supply to the body is reduced and the performance of those affected is drastically limited. In advanced stages, pulmonary hypertension can develop into a life-threatening condition.

The diagnosis of idiopathic pulmonary hypertension is very rare. Much more often, PH is the result of a pre-existing condition, such as left heart disease or lung disease and/or lack of oxygen (hypoxia). Symptoms of PH include shortness of breath, weakness, severe circulatory problems and eventually right heart failure. Patients who develop PH usually die of right cardiac decompensation.

Only a few drugs are currently approved for the treatment of pulmonary hypertension. Agents include prostanoids, sGC stimulators (riociguat), endothelin receptor antagonists and phosphodiesterase-5 inhibitors. However, the effectiveness of the drugs is low and the medications usually do not lead to a cure, but only delay the progression of the disease process. In addition, considerable side effects can occur, so that a lung transplantation is sometimes necessary instead of drug therapy. Importantly, these drugs are at present only approved for the relatively rare case of pulmonary arterial hypertension (including idiopathic pulmonary hypertension), whereas no pharmacological therapies are presently approved for treatment of the most frequent forms of PH, namely PH associated with left heart disease or lung disease and/or lack of oxygen.

"Pulmonary arterial hypertension" (PAH) is a relevant subgroup according to the most recent WHO classification system (WHO Group I).

Accordingly, the objective of the present invention is to provide an effective medical agent for treatment and prevention of pulmonary hypertension, in particular of pulmonary arterial hypertension (WHO Group I). Furthermore, the medical agent should have acceptable side-effect profile when being used for the treatment of pulmonary hypertension.

### GENERAL DESCRIPTION OF THE INVENTION:

The present invention is directed to subject matter as defined in the claims and as set out in detail below.

It has been surprisingly found that a pharmaceutically acceptable lithium salt can be used in the treatment or prevention of pulmonary hypertension.

Lithium compounds, also known as lithium salts, have been used as a psychiatric medication. They are primarily used to treat bipolar disorder and treat major depressive disorder that does not improve following the use of antidepressants. In these disorders, it reduces the risk of suicide.

In the context of the invention, it was surprisingly found that lithium salts are also suitable for treating and/or preventing pulmonary hypertension, thereby extending the therapeutical spectrum of lithium salts.

Advantageously, since lithium salts are used to treat depression, schizophrenia, bipolar disorder and cluster headaches are clinically approved. Since lithium salts are clinically approved and show only limited side-effects for the treatment of said neurological and psychiatric diseases, lithium salts can also be expected to show an acceptable side-effect profile in the treatment of pulmonary hypertension.

The present invention demonstrates that the use of lithium compound, i.e. lithium salts, can be extended to further medical indications, namely pulmonary hypertension.

According to the present invention, lithium salt is used for treatment or prevention of pulmonary hypertension; preferably pulmonary arterial hypertension (WHO Group I). Of note, the invention is directed to the indication of lithium salts for the treatment of pulmonary hypertension in general, i.e. it is applicable to all WHO groups, but there is a particular preference for WHO group I.

It has surprisingly been found that administration of lithium salts is particularly suitable for the treatment of pulmonary artery stiffness in PH.

Lithium salts for use of the present invention are preferably in the form of pharmaceutically acceptable salt. Such pharmaceutically acceptable salts are suitable for pharmaceutical application, in particular regarding quality and purity.

Preferably, the lithium salt is used for prevention of pulmonary hypertension in patients with an elevated risk for pulmonary arterial hypertension (WHO Group I), for instance with BMPR2-Mutations.

The pharmaceutically acceptable lithium salt is selected preferably from lithium carbonate, lithium sulfate and lithium citrate. Alternatively, lithium chloride may be used.

Salts may be prepared by exchanging a counter-ion of a compound of the invention in the form of a salt with another counter-ion, for example using a suitable ion exchange resin. The salt form may confer improved pharmacokinetic properties. The pharmaceutically acceptable salt may also positively affect the pharmacodynamics of the compound with respect to its therapeutic activity in the body. An example of pharmacodynamics property that may be favorably affected is the manner in which the compound is transported across cell membranes, which in turn may directly and positively affect the adsorption, distribution, biotransformation and excretion of the compound.

The present invention also relates to a pharmaceutical composition comprising as an active ingredient lithium salts for use in treatment and/or prevention of a disease, e.g. of pulmonary hypertension; preferably pulmonary arterial hypertension (WHO Group I).

Pulmonary arterial hypertension (PAH) is a particular form of the broader condition pulmonary hypertension, which is high blood pressure in the lungs. In PAH, this increased pressure in the vessels can e.g. be caused by obstruction in the small arteries in the lung for a variety of reasons. The lithium salt is generally for the treatment and/or prevention of pulmonary hypertension. In particular is it is for the treatment and/or prevention of pulmonary arterial hypertension; PAH according to WHO Group I.

Such pharmaceutical composition may comprise, in addition to lithium salts, one or more pharmaceutically acceptable excipients. The term "excipient" is used herein to describe any ingredient other than the compound of the invention. The choice of excipient will to a large extent depend on the particular mode of administration. Excipients can e.g. be suitable carriers, retardants, boosters, prolonging substances, adjuvants, stabilizers, binders, emulsifiers, surface active agents, penetration enhancers suspending agents, disintegrants, buffers, additional salts, dilutents, solvents, dispersion media, fillers, lubricants, propellants, preservatives, flavours or mixtures thereof.

The pharmaceutical composition for use according to the present invention preferably comprises the lithium salt combined with a delivery vehicle. The delivery vehicle is any compound or composition suitable for delivering the biologically active lithium salts or the pharmaceutically acceptable salt thereof to a location within the body, where the biological activity is needed or desired. Examples of suitable delivery vehicles comprise microparticle, nanoparticle, hydrogel, perivascular drug delivery vehicle, endovascular drug delivery vehicle, a stent or a combination thereof.

Oral application is one advantageous way of administration as it is simple and already approved for clinical use in psychiatry. On the other hand, more targeted approaches, as for instance using a delivery vehicle, can help to reduce adverse effects of lithium therapy.

Perivascular delivery vehicle technologies suitable for use in the present invention are generally known to those of skill in the art, and, thus, need not be explained at length herein. For instance, exemplary known perivascular drug delivery technologies include those described by Chen, et al. (U.S. Patent Application Publication No. 2005/0079202) and Nathan (U.S. Patent Application Publication No. 2003/0228364). These exemplary perivascular delivery systems include a polymeric delivery vehicle that can be injected or directly placed, for instance via surgery, at a particular location so as to provide controlled release of the lithium salts encapsulated or otherwise loaded therein over a period of time.

Many endovascular delivery vehicles are likewise known in the art. For example, DiCarlo, et al. (U.S. Patent No. 6,929,626) describe an intraluminally placeable tubular device that can be located within the lumen of a blood vessel and coated or otherwise loaded with a drug, e.g., the lithium salts described herein. The tubular member includes yarns interconnected in a pattern defining opposed interior and exterior textile surfaces. At least one of the textile surfaces is the body fluid-contacting luminal surface or the body lumen-contacting exterior surface. Wu, et al. (U.S. Patent No. 6,979,347) describe an apparatus and associated method for delivering a therapeutic substance such as the lithium salts of the present invention, to a vascular lumen. Specifically, an implantable prosthesis, such as a stent, can be utilized that has grooves or trenches formed thereon. The grooves are formed on specific regions of the stent struts to increase the flexibility of the stent. The grooves also provide a location for carrying the lithium salts for delivery from the device following implantation. For example, the lithium salts, or a pharmaceutical composition thereof, can be deposited directly into the grooves using conventional spray or modified dip techniques.

In another embodiment, the pharmaceutical composition of the invention comprising a lithium salt can be administered by use of a hydrogel delivery vehicle. Hydrogels are herein defined to include polymeric matrices that can be highly hydrated while maintaining structural stability. Suitable hydrogel matrices can include un-crosslinked and crosslinked hydrogels. In addition, crosslinked hydrogel delivery vehicles of the invention can optionally include hydrolyzable portions, such that the matrix can be degradable when utilized in an aqueous environment, e.g., in vivo. For example, the delivery vehicle can include a cross-linked hydrogel including a hydrolyzable cross-linking agent, such as polylactic acid, and can be degradable in vivo. Hydrogel delivery vehicles of the present invention can include natural polymers such as glycosaminoglycans, polysaccharides, proteins, and the like, as well as synthetic polymers, as are generally known in the art. A nonlimiting list of hydrophilic polymeric materials that can be utilized in forming hydrogels of the present invention can include dextran, hyaluronic acid, chitin, heparin, collagen, elastin, keratin, albumin, polymers and copolymers of lactic acid, glycolic acid, carboxymethyl cellulose, polyacrylates, polymethacrylates, epoxides, silicones, polyols such as polypropylene glycol, polyvinyl alcohol and polyethylene glycol and their derivatives, alginates such as sodium alginate or crosslinked alginate gum, polycaprolactone, polyanhydride, pectin, gelatin, crosslinked proteins peptides and polysaccharides, and the like.

The delivery vehicles of the present invention can include a combination of one or more delivery vehicles. For instance, a hydrogel delivery vehicle can be combined with a patch, a stent, a perforated balloon, a vascular graft, or any other suitable device, for delivery of the disclosed agents to connective tissue.

In a preferred embodiment, the delivery vehicle comprises or consists of a microparticle or a nanoparticle. Particularly suitable micro- and nanoparticles are disclosed in US 2014/0017263 A1.

In general, any bulk biocompatible material capable of being formed to a useful size can be utilized in forming the micro- or nanoparticles of the delivery vehicles. In one embodiment, a polymeric particle can be utilized. For instance, particles formed from polystyrene, poly(lactic acid), polyketal, butadiene styrene, styrene-acrylic-vinyl terpolymer, poly(methyl methacrylate), poly(ethyl methacrylate), poly(alkyl cyanoacrylate), styrene-maleic anhydride copolymer, poly(vinyl acetate), poly(vinyl pyridine), poly(divinylbenzene), poly(butylene terephthalate), acrylonitrile, vinyl chloride-acrylates, poly(ethylene glycol), and the like, or an aldehyde, carboxyl, amino, hydroxyl, or hydrazide derivative thereof can be utilized.

Particles formed of biological polymers such as proteins can be used. For instance, particles formed of albumin, dextran, gelatin, chitosan, etc. can be utilized, preferably albumin is used for forming particles. Such particles can be preferred as they can be formed without the use of organic solvents according to known methods.

Other biocompatible materials as may be utilized in forming disclosed particles can include, without limitation, oxides such as silica, titania, zirconia, and the like, and noble metals such as gold, silver, platinum, palladium, and the like. In general, the materials will be biocompatible and nonimmunogenic.

The particles can be biodegradable. For instance, biodegradable polymeric particles formed from polysaccharide and/or poly(lactic acid) homopolymers and copolymers can be used. For example, particles formed of poly(lactic-co-glycolic acid) (PLGA) copolymers and derivatives thereof can be utilized. In one embodiment, a poly(ethylene glycol) (PEG)/poly(lactic acid) (PLA) block copolymer can be utilized in forming the particles. A PEG-PLA block copolymer is an amphiphilic polymer with good stability in vivo. With good biocompatibility, the PEG hydrophilic component of the block copolymer can increase the solubility of insoluble compounds, prevent protein absorption on the particle surface, make the particles unrecognizable by the reticuloendothelial system as foreign bodies, and thereby provide particles that can have a characteristic of long circulation.

Selection of bulk nanoparticle material can be utilized to provide primary control of release rate of a biologically active compound from the loaded particle. For instance, selection of a biodegradable material can be utilized to increase the rate of compound release and provide a release mechanism that can be limited to a large extent by nanoparticle degradation rate and to a lesser extent by diffusion of the active compound from the bulk nanoparticle. Alternatively, materials can be utilized such that active compound release rate is limited by only diffusion (e.g., a nondegradable particle) or nanoparticle degradation rate (e.g., essentially no diffusion of the active compound through the particle due to small matrix mesh size).

As mentioned, the particles of the delivery agents can be microparticles or nanoparticles. By way of example, the size, i.e., the average diameter of formed nanoparticles can generally be less than about 500 nanometers, for instance less than about 200 nm, or less than about 100 nm. In one particular embodiment, the nanoparticles can be less than about 50 nm in size, for instance about 20 nm in average diameter. In one embodiment, nanoparticles can be formed having an average diameter of between about 50 nm and about 400 nm, or between about 100 nm and about 300 nm. In one embodiment, the nanoparticles can have an average diameter of about 200 nm.

Larger particles can alternatively be formed. For instance, in other embodiments, microparticles can be formed having a size of up to about 50 micrometers (µm). In general, the preferred size of the particles can depend upon the specific application, e.g., the specific method of delivery of the agents such as via surface application (as in a cream or lotion), via parenteral injection using the respiratory or digestive tract, etc., as well as the desired release rate of a treatment compound from the particles. For instance, in one embodiment, the particles can be of a size to prevent cellular uptake so as to remain in the extracellular matrix and available for interaction with damaged elastic fibers. Thus, the particles may be larger than about 100 nm in one embodiment, as smaller particles have been shown to exhibit higher cellular uptake. Particles can also be small enough so as to penetrate endothelium and penetrate basement membrane so as to contact the elastin fibers of the connective tissue. For instance, particles can be less than about 400 nm in average diameter in one embodiment so as to penetrate endothelium and basement membrane.

Generally, the particles are substantially spherical in shape, although other shapes including, but not limited to, plates, rods, bars, irregular shapes, etc., are suitable for use. As will be appreciated by those skilled in the art, the composition, shape, size, and/or density of the particles may vary widely.

Particles can be designed with a desirable surface charge so as to better target damaged elastin. For instance, positively charged nanoparticles have shown superior cellular uptake as compared to negatively charged particles. Thus, in one embodiment, particles can be developed with a negative surface charge to maintain the particles in the extracellular matrix and avoid cellular uptake.

Disclosed particles can be loaded with one or more biologically active compounds according to any suitable method. For instance, a precipitation method can be utilized to form the loaded particles in a one-step formation process. According to this method, a particle bulk material (e.g., a biocompatible polymer such as poly-(D,L-lactide-co-glycolide or a PGA/PLA copolymer) can be dissolved in a solvent to form a first solution. Suitable solvents can depend upon the specific materials involved. For example, organic solvents including acetone, tetrahydrofuran, dimethylsulfoxide, dimethylformamide, or acetonitrile and the like can be utilized. This first solution can undergo standard processing such as sonication, etc., so as to adequately solubilize the polymer. The first solution can then be added, generally dropwise, to a second solution. The second solution can be, e.g., an aqueous solution. Either spontaneously or following an emulsification method, for instance following sonication, particles can form that include the polymer bulk material.

According to a single-step formation process, a biologically active compound (i.e.., the lithium salt) can be included in either the first solution or the second solution. Upon formation of the particles, the biologically active compound, i.e. the lithium salt can be incorporated in the particles with the polymer bulk material.

The initial concentration of lithium salts within or on a particle can vary. For example, in one embodiment, loading concentration of biologically active compound like e.g. lithium salts in a particle can vary from about 4 wt. % to greater than about 40 wt. % by weight of the particle, with higher and lower concentrations possible depending upon specific compound, particle bulk material, and the like. For instance, in an embodiment in which a biologically active compound exhibits high solubility in the bulk particle material, a very high loading level can be attained, particularly when both materials are highly hydrophobic.

Precipitation methods can be useful, as such methods can provide monodisperse polymer particles loaded with a biologically active compound like e.g. lithium salts. Moreover, precipitation formation processes can be adjusted according to processing methods known to those skilled in the art to provide particles of a desired size and including a desired concentration of biologically active compound. For instance, modification of particle size can be obtained through modification of the concentration and/or type of surfactant included in the receiving solution according to known practices.

Formation processes can include two step processes in which particles are first formed followed by a second loading step in which lithium salts or other additional active agents are loaded into the formed particles. For instance, a method can include swelling a pre-formed, crosslinked polymeric particle in a solution that includes the biologically active compound so as to load the particle via a diffusion process. In another embodiment, loading method can include double emulsion polymerization, which enables loading of hydrophilic compounds into hydrophobic particles.

Methods of forming particles loaded with a biologically active compound like e.g. lithium salts are not limited to the precipitation method. Other micro- and nanoparticle formation processes as are known in the art as can be utilized in forming particles loaded with active compounds. For instance, supercritical fluid processing methods as disclosed by U.S. Pat. No. 7,754,243 to Sun can be utilized to form extremely small nanoparticles, e.g., less than about 20 nm having a very narrow size distribution and little or no particles that do not include the biologically active compound in the as-formed suspension.

Loaded particles can be formed so as to control the rate of release of active compound from a particle. Suitable control mechanisms are known to those of skill in the art. For instance, release rates can depend upon the relative concentration of active compound to bulk particle material, upon the molecular weight and degradation characteristics of the bulk nanoparticle material, upon the mesh size of a polymer particle matrix, upon the binding mechanism between the surface of a particle and an active compound, and so forth, as is known. In any of these cases, one of ordinary skill in the art is capable of engineering a system so to achieve desirable release rate. For instance, in the case of purely diffusion-limited release, such control can be achieved by variation of compound concentration within particles and/or particle size, particle polymer mesh size, and so forth. In the case of purely degradation-limited release, polymer monomer units, for instance glycolic acid content of a PLGA polymer, and/or molecular weight of particle bulk material, as well as particle size, can be adjusted to "fine tune" active compound release rate. For example, use of PLGA polymers with higher glycolic acid content and lower molecular weight can lead to an increased degradation rate of a particle formed with the polymer. Release rate of active compound from particles can be adjusted utilizing the above parameters so as to produce carriers capable of sustained release for periods varying from a few days to a few months, with the maximum release rates generally varying from a few hours to a few weeks.

According to another embodiment, release rate of an active compound can be controlled through binding, generally noncovalent binding, of the active compound to a ligand within the particle.

Exemplary methods and materials of as can be utilized in one embodiment are described in U.S. Pat. No. 8,128,952 to Metters, et al., which is incorporated herein by reference. According to this method, a ligand can be selected that has an affinity for the biologically active compound to be delivered by the agent. For instance, a ligand can be selected according to a predetermined dissociation constant (K_{D}) describing this affinity and the ligand can be incorporated into the particle at a predetermined concentration level. The rate of release of the active compound from the particle that is established upon incorporation of the compound into the particle can then be controlled according to these particular parameters, i.e., K_{D} and the concentration of the ligand.

Biologically active compounds, like lithium salts, need not necessarily be incorporated within the bulk particle material. For example, in another embodiment, a biologically active compound can be bound to the surface of a particle. For example, a compound can be bound to the surface of a particle utilizing chemistry similar to that as is described in more detail below with regard to the binding of the targeting antibodies to the particles, e.g., via glutaraldehyde crosslinking.

Delivery vehicles can include additional materials on or within the particles, in addition to lithium salts or more active compounds that can treat elastic fiber degradation. Such materials can be active materials, providing direct benefit to the tissue in addition to the stabilization provided by the biologically active compound, or may be supporting materials, improving delivery, compatibility, or reactivity of other materials in the delivery agent. For example, in one embodiment, the delivery vehicle can include glutaraldehyde. Glutaraldehyde, when targeted to connective tissue, can form covalent cross-links between free amines in proteins in order to further stabilize the tissue.

In addition to the particle and lithium salts (and optionally further biologically active compounds), the delivery vehicle preferably includes an anchoring agent that binds at or near degraded elastic fibers so as to provide the lithium salts at the targeted site. Preferably, the anchoring agent binds specifically to a structure associated with a pulmonary blood vessel; more preferably to a structure of a cell of a pulmonary blood vessel or a component of the extracellular matrix of a pulmonary blood vessel. Preferably, the loaded particle can be coated with an anchoring agent that can specifically bind elastin, preferably human elastin, as degraded elastic fibers will include elastin exposed due to degradation of the microfiber scaffolding. Accordingly, in one embodiment, a delivery vehicle can include at a surface an antibody or a fragment thereof that is specific for elastin for targeting the vehicle to degraded elastic fibers and providing the lithium salts of the delivery vehicle to the damaged elastic fibers at the anchoring site.

The anchoring agent bound to a surface of a particle can be a polypeptide, e.g., either a complete protein or a fragment thereof, that can recognize and bind receptors at the targeted site. This is not a requirement of the disclosed anchoring agents, however, and in another embodiment, an anchoring mechanism can utilize a non-proteinaceous anchoring agent, for instance a polysaccharide that can bind a particle to a targeted location, e.g., elastin exposed due to degradation of the elastic fiber.

Anchoring agents can be natural or synthetic agents. The anchoring agent can include polyclonal or monoclonal antibodies as desired. Antibodies can be raised according to known methods. Preferably, the anchoring agent is an antibody or an antigen binding fragment thereof.

As used herein, the term "antibody" refers to single chain, two-chain, and multi-chain proteins and glycoproteins that belong to the classes of polyclonal, monoclonal, chimeric and human or humanized immunoglobulin proteins. The term "antibody" also includes synthetic and genetically engineered variants thereof.

As used herein, the term "antibody fragment" or "antigen binding fragment" of an antibody refers to a Fab fragment, a Fab' fragment, a F(ab')2 fragment, a F(ab')3 fragment, a Fd fragment, a Fd' fragment, a Fv fragment, a scFv, a bivalent scFv, a diabody, a linear antibody, single chain antibodies, functional heavy chain antibodies (nanobodies), as well as any portion of an antibody having specificity toward at least one desired epitope, that competes with the intact antibody for specific binding (e.g., an isolated portion of a complementarity determining region having sufficient framework sequences so as to bind specifically to an epitope). Antigen binding fragments can be produced by recombinant techniques, or by enzymatic or chemical cleavage of an intact antibody.

As used herein, the term "human antibody" refers to an antibody that possesses a sequence that is derived from a human germ-line immunoglobulin sequence, such as antibodies derived from transgenic mice having human immunoglobulin genes (e.g., XENOMOUSE ^{™}genetically engineered mice (Abgenix)), human phage display libraries, in cows (milk) or human B cells.

As used herein, the term "humanized antibody" refers to an antibody that is derived from a non-human antibody (e.g., murine) that retains or substantially retains the antigen-binding properties of the parent antibody but is less immunogenic in humans. Humanized as used herein is intended to include deimmunized antibodies.

The term "modified" or "recombinant" antibody, as used herein, refers to antibodies that are prepared, expressed, created or isolated by recombinant means, such as antibodies expressed using a recombinant expression vector transfected into a host cell, antibodies isolated from a recombinant, combinatorial antibody library, antibodies isolated from an animal (e.g., a mouse) that is transgenic for human immunoglobulin genes or antibodies prepared, expressed, created or isolated by any other means that involves splicing of human immunoglobulin gene sequences to other DNA sequences. Such modified antibodies include humanized, CDR grafted, chimeric, *in vitro* generated (e.g., by phage display) antibodies, and may optionally include variable or constant regions derived from human germline immunoglobulin sequences or human immunoglobulin genes or antibodies which have been prepared, expressed, created or isolated by any means that involves splicing of human immunoglobulin gene sequences to alternative immunoglobulin sequences.

The term "monospecific antibody" refers to an antibody that displays a single binding specificity and affinity for a particular target, e.g., epitope. This term includes a "monoclonal antibody" or "monoclonal antibody composition," which as used herein refer to a preparation of antibodies or fragments thereof of single molecular composition.

The term "bispecific antibody" or "bifunctional antibody" refers to an antibody that displays dual binding specificity for two epitopes, where each binding site differs and recognizes a different epitope.

In a preferred embodiment, the antibody or the antigen binding fragment thereof to be used as anchoring agent specifically binds to elastin, preferably to human elastin.

Following formation, the anchoring agent can be further processed so as to facilitate conjugation with the particle of the delivery vehicle. For example, following initial formation of the anchoring agent, e.g., an antibody, the antibody can be further processed so as to be more readily bound to the particle of the delivery agent. For instance, antibody can be reacted with a thiolation compound such as Traut's Reagent (2-iminothiolane) to produce a thiolated antibody.

The antibody can be conjugated with the particle according to any suitable process. For example, a particle can include surface reactive groups to facilitate conjugation of the particle with an anchoring agent, e.g., an antibody. Surface reactive groups can include, without limitation, aldehyde, carboxyl, amino, hydroxyl, and the like. Surface reactive groups can either exist on the particle surface as formed or can be added to the surface following formation, for instance via oxidation, amination, etc., of the formed particle, as is generally known in the art. The antibody can then be conjugated with the particle, for instance through reaction with maleimide in the illustrated embodiment in which the antibody is a thiolated antibody as described above.

Anchoring agents can be attached to a particle via either nonspecific adsorption or a covalent bond. Preferred attachment methods can generally depend upon the desired application of the formed conjugates. For instance, in those embodiments in which a system is designed to function in vivo, the particles can be expected to encounter multiple collisions with various biological agents and tissues. Accordingly, covalent binding can be preferred in such an embodiment, to better ensure that the anchoring agents will not be dislodged through collision of the particles with other materials.

The specific chemistry utilized to bind the anchoring agent (and optionally the biologically active compound like e.g. lithium salts) to the particle surface is not particularly limited. For example, in one embodiment, a proteinaceous anchoring agent can be bound to a chloromethylated particle according to a nucleophilic substitution reaction between a protein amine group and the alkyl chloride of the particle. In another embodiment, soluble carbodiimide (EDC) and glutaraldehyde chemistry can be used to achieve covalent binding of amine groups of a proteinaceous agent to carboxylated and aminated particles, respectively. According to yet another embodiment, a proteinaceous agent can be bound to a particle through initial covalent attachment of a streptavidin monolayer to a particle followed by controllable attachment of desired amounts of biotinylated proteins. The presence of a streptavidin monolayer can also eliminate potential problems related to direct interaction of functional proteins with particles in the environment in which the particles will be utilized. According to yet another embodiment, a proteinaceous anchoring agent can be covalently attached to a particle using a crosslinking agent, for instance a phenylazide crosslinking agent such as sulfo-HSAB (N-Hydroxysulfosuccinimidyl-4-azidobenoate) a photoreactive reagent available from Pierce, Inc. that can crosslink amine groups of the proteinaceous anchoring agent and C-H or C-C bonds of a polymeric particle.

In one embodiment, a molecular spacer, for instance a hydrophilic spacer, can be utilized to tether an anchoring agent to a particle. Utilization of a spacer can prevent interaction of covalently bound anchoring agents, e.g., proteins, with the particle surface and thus prevent structural changes of the protein that can lead to partial or complete loss of functionality of the protein. Spacers can include long (e.g., weight average molecular weight between about 2,000 and about 20,000 Da) hydrophilic polymers so as to minimize interaction of the bound proteins with the surface of the particle. Hydrophilic spacers can include, without limitation, poly(ethylene glycol), polyvinyl alcohol, polysaccharides, and so forth.

In an exemplary method of attachment of a proteinaceous anchoring agent to a particle through a poly(ethylene glycol) (PEG) spacer, the PEG spacer and the particle can include or be processed to include functionality so as to facilitate binding to one another. For example, the PEG spacer can include aldehyde functionality and can bind to the aminated particle through covalent reaction between the aldehyde group of the spacer and the amine group of the particle. The thiolated antibody can then be attached to the spacer according to a simple process including mixing of a solution including the thiolated antibody with an aqueous suspension of particles in the presence of maleimide so as to form the delivery agent.

At the final stage of conjugation, a particle can be blocked, for instance, with a surfactant, such as Tween^{®} 20, Pluronic^{®}, or dextrane that can be adsorbed on the particle to block any hydrophobic surface exposed to the solution as well as to displace any noncovalently bound agents. Low concentrations of such materials generally do not interfere with the activity of agents such as water-soluble enzymes. The presence of a surfactant can reduce undesirable protein-particle interactions and prevent particle aggregation. It can also prevent nonselective "fouling" of the surface of a particle with other proteins in the environment in which the material is utilized that could potentially deactivate a system.

As previously mentioned, surface sites of a particle to which an anchoring agent can be bound can vary. For instance, in one embodiment, carboxyl-modified particles can be utilized. For example, carboxyl-modified PLA-based particles can be utilized. According to one such embodiment, an NH₂-PEG-COOH spacer can be bound to a particle via the amine group using carbodiimide chemistry according to known methodology. An anchoring agent can then be likewise coupled to the carboxyl group of the spacer using carbodiimide chemistry. The surface of the particle can then be blocked with a suitable agent (e.g., Tween^{®} 20, Pluronic^{®}, dextran, and the like), as described above.

Beneficially, it is possible to precisely engineer a particle to exhibit anchoring properties for a desired application. For example, the binding capacity and length of time a particle can remain bound to the targeted tissue, e.g., damaged vasculature, can be engineered by altering particle size, type of the anchoring agent, and/or anchoring agent concentration on the particle surface.

According to one embodiment, a single anchoring agent can bind to more than one particle. For instance, in those embodiments in which an anchoring agent can be bound to a particle via amine groups of the protein, and as protein molecules have more than one amine group, a single protein molecule can potentially bind to more than one particle. This may result in the formation of dimers and larger aggregates of particles. While formation of large aggregates may be preferred in some embodiments, for instance in some in vitro assay applications or in an in vivo topical application embodiment in other applications it can be preferable to minimize aggregation. Accordingly, in one embodiment, a lower particle concentration and/or a higher concentration of surfactant, as well as variation in surfactants, can be utilized during a formation process in order to minimize aggregation of particles.

Of note, the invention is directed to the indication of lithium salts for the treatment of pulmonary hypertension in general, i.e. it is applicable to all WHO groups, but there is a preference for WHO group I.In accordance with the present invention, lithium salts or a pharmaceutically acceptable salt thereof or the pharmaceutical composition of the invention is used in treatment and/or prevention of a disease, e.g. of pulmonary hypertension; preferably the pulmonary arterial hypertension (WHO Group I).

According to WHO classification, pulmonary hypertension (PH) is divided into 5 separate groups, wherein Group I (pulmonary arterial hypertension) is further subdivided into Group I' and Group I" classes.

The most recent WHO classification system can be summarized as follows:
WHO Group I - pulmonary arterial hypertension (PAH);
WHO Group I' - pulmonary veno-occlusive disease (PVOD), pulmonary capillary hemangiomatosis (PCH);
WHO Group I" - persistent pulmonary hypertension of the newborn;
WHO Group II - pulmonary hypertension secondary to left heart disease;
WHO Group III - pulmonary hypertension secondary to lung disease like e.g. chronic hypoxia, chronic obstructive pulmonary disease (COPD), interstitial lung disease, mixed restrictive and obstructive pattern pulmonary diseases, sleep-disordered breathing, alveolar hypoventilation disorders, chronic exposure to high altitude, and/or developmental abnormalities;
WHO Group IV - chronic arterial obstruction;
WHO Group V - pulmonary hypertension with unclear or multifactorial mechanisms.

Preferably, lithium salts or a pharmaceutically acceptable salt thereof or the pharmaceutical composition of the invention are used in treatment and/or prevention of pulmonary arterial hypertension (WHO Group I).

As used herein, the term "treating" encompasses reversing, alleviating or inhibiting the progress of a disease, disorder or condition, or improvement of one or more symptoms of such disease, disorder or condition, to which such term applies. As used herein, "treating" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein the term "treating" may also refer to preventing the recurrence of a disease, disorder or condition or of one or more symptoms associated with such disease, disorder or condition. The terms "treatment" and "therapeutically," as used herein, refer to the act of treating, as "treating" is defined above.

For the purpose of the present invention any reference to a method for treatment comprising the administration of a compound or to a use of a compound in a method of manufacture of a medicament for treatment of a disease is to be understood as a reference to said compound for use in such a method.

The present invention also relates to lithium salts or a pharmaceutically acceptable salt thereof or a pharmaceutical composition for use in manufacture of a medicament for treatment and/or prevention of pulmonary hypertension. Preferably, the pulmonary hypertension is secondary pulmonary hypertension; more preferably the pulmonary hypertension is pulmonary hypertension secondary to left heart disease (LHD; WHO group II).

The present invention also relates to a method of treatment of pulmonary hypertension, wherein a patient in need of such therapy is administered a therapeutically effective dose of lithium salts or a pharmaceutical composition comprising the same. Preferably, the pulmonary hypertension is secondary pulmonary hypertension; more preferably the pulmonary hypertension is pulmonary hypertension secondary to left heart disease (LHD; WHO group II).

The lithium salts or the pharmaceutically acceptable salt thereof of the pharmaceutical composition of the invention are administered preferably at an effective dose. An "effective dose" is the dose of lithium salts that upon administration to a patient yields a measurable therapeutic effect with regard to the disease of interest. In the present invention an effective dose is the dose of lithium salts that upon administration to a patient yields a therapeutic effect with regard to at least one disease related symptom in a patient or patients suffering from said disease.

Preferably, the orallithium salts according to the present invention are administered at a dose of 1 mg/kg/d to 300 mg/kg/d, preferably of 20 mg/kg/d to 150 mg/kg/d. In any event, the physician or the skilled person will be able to determine the actual dose which will be suitable for an individual patient, which is likely to vary with the age, weight, sex, and concomitant illnesses such as renal or hepatic dysfunction and response of the particular patient to be treated. The above-mentioned dosages are exemplary of the average case. There can, of course, be individual instances where higher or lower dosage ranges are appropriate, and such are within the scope of the invention.

In particular, a dosage of 15 to 60 mg/kg/d is considered as particularly preferable for the medical indication according to the present invention.

In order to minimize drug reactions, a lower dosage should be used initially. The serum lithium levels should be twice weekly monitored 12 hours after dose until they stabilize. When stable, it is sufficient to monitor serum levels every other month. Stable levels of 0.5-.1 mmol/l should be envisaged and should not exceed 1.5 mmol/l.

Therefore, according to a preferred embodiment, the lithium salt is for being administered such that the serum level concentration of lithium in the range of 0,5 to 1,5.

According to an even more preferred embodiment, the lithium salt is for being administered in such a way that a serum level concentration of lithium in the range of 0,5 to 1,5 once the serum concentration has been stabilized.

The lithium salts or pharmaceutically acceptable salt thereof or the pharmaceutical composition for use of the present invention are preferably administered orally, intravenously, subcutaneously, bucally, rectally, dermally, nasally, tracheally, bronchially or by any other parenteral route or via inhalation in a pharmaceutically acceptable dosage form. The pharmaceutical composition for use according to the invention is configured for e.g. topical or systemic administration, preferably for intravascular, intravenous, intra-arterial, intracardial, intra-pulmonal and/or nasal administration.

The lithium salts or pharmaceutically acceptable salt thereof or the pharmaceutical composition for use of the present invention may be administered orally. Oral administration may involve swallowing, so that the compound enters the gastrointestinal tract, or buccal or sublingual administration may be employed by which the compound enters the blood stream directly from the mouth.

Oral application is an advantageous way of administration as it is simple and, in particular, already approved for clinical use in psychiatry.

Formulations suitable for oral administration include: solid formulations such as tablets; capsules containing particulates, liquids, or powders; lozenges (including liquid-filled); and chews; multi- and nano-particulates; gels; solid solutions; liposomes; films, ovules, sprays and liquid formulations. Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

For tablet dosage forms, depending on dose, the compound may make up from 1 weight % to 80 weight % of the dosage form, more typically from 5 weight % to 60 weight % of the dosage form. In addition to the compound, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pre-gelatinized starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% compound, from about 10 weight % to about 90 weight % binder, from about 0 weight % to about 85 weight % diluent, from about 2 weight % to about 10 weight % disintegrant, and from about 0.25 weight % to about 10 weight % lubricant.

The lithium salts or the pharmaceutical composition for use of the present invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as additional salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water. The preparation of parenteral formulations under sterile conditions, for example, by lyophilization, may readily be accomplished using standard pharmaceutical techniques well known to those skilled in the art.

The lithium salts or the pharmaceutical composition for use of the present invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Liposomes may also be used. Typical carriers include alcohol, water, mineral oil, liquid petrolatum, white petrolatum, glycerin, polyethylene glycol and propylene glycol.

The lithium salts or the pharmaceutical composition for use of the present invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomizer (preferably an atomizer using electro-hydrodynamics to produce a fine mist), or nebulizer, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin. The pressurized container, pump, spray, atomizer, or nebulizer contains a solution or suspension of the 4,5-diarylimidazole derivatives of the invention comprising, for example, ethanol, aqueous ethanol, or a suitable alternative agent for dispersing, solubilizing, or extending release of the active, a propellant(s) as solvent and an optional surfactant, such as sorbitan trioleate, oleic acid, or an oligolactic acid.

The use of lithium salts or the pharmaceutical composition in the treatment of pulmonary hypertension, preferably of pulmonary hypertension secondary to left heart disease, may have the advantage that such compound may be more efficacious than, be less toxic than, be longer acting than, be more potent than, produce fewer side effects than, be more easily absorbable than, have better pharmacokinetic profile (e.g. higher oral bioavailability and/or lower clearance) than, and/or have other useful pharmacological, physical, or chemical properties over compounds known in the prior art for treatment of said diseases.

### EXPERIMENTAL DATA

The Examples of the invention as shown in the Figures provide evidence that a pharmaceutically acceptable lithium salt can be used in the treatment or prevention of pulmonary hypertension.

According to the pathogenesis, pulmonary hypertension is shown in five divided into groups. In all groups there is a reorganization of the histological level pulmonary vascular structure and muscularization of small precapillary arterioles with simultaneous hyperplasia and hypertrophy of pulmonary artery smooth muscle cells (PASMCs). The hyperplasia is characterized by a dedifferentiation and increased proliferation of the PASMCs, while the hypertrophy is characterized by an increased protein production. Through these cellular and vascular changes, the pulmonary vascular resistance increases, so that pulmonary hypertension develops as a result. Due to their prominent role in PH, experiments were carried out inter alia on PASMCs.

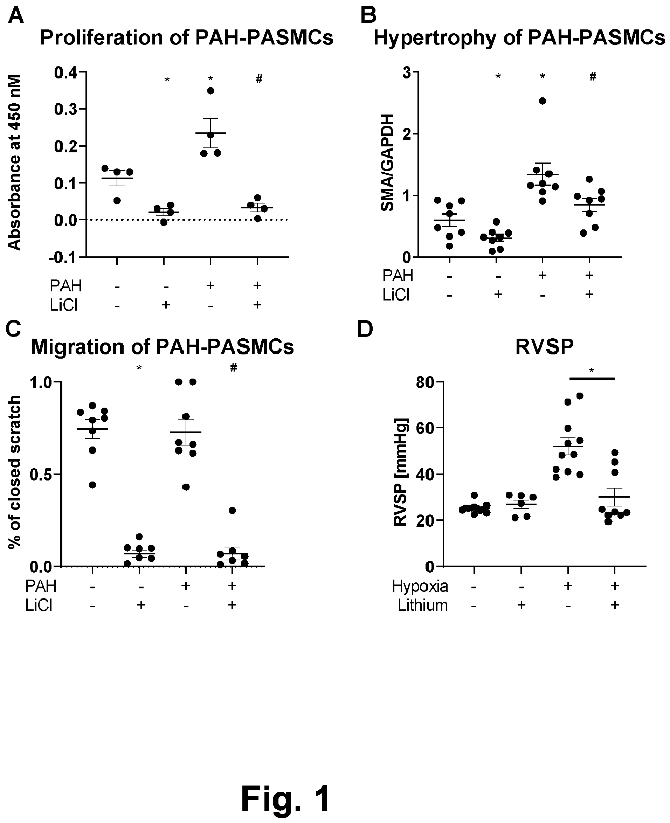

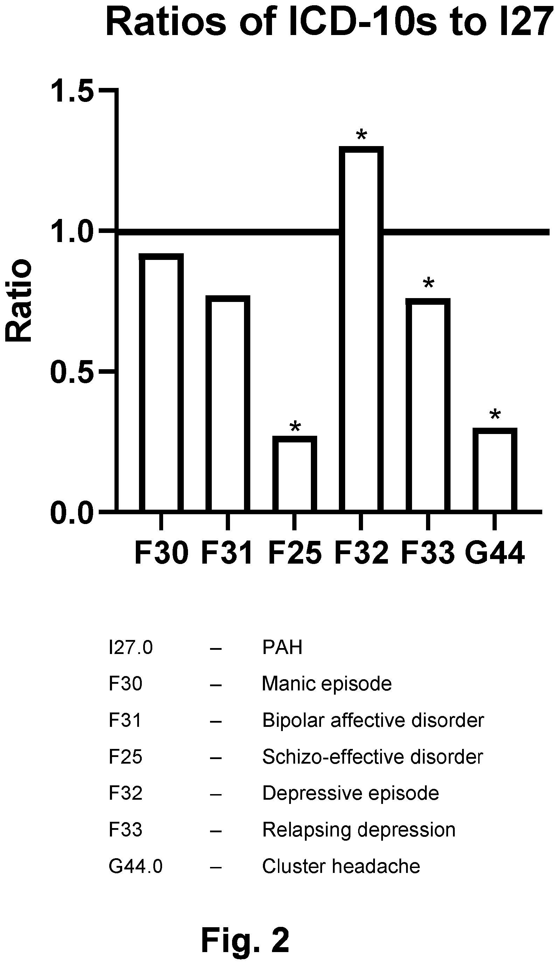

Fig. 1 and Fig. 2 demonstrate the effect of Lithium on pulmonary hypertension, thereby illustrating the use of lithium salts in the treatment or prevention of pulmonary hypertension.

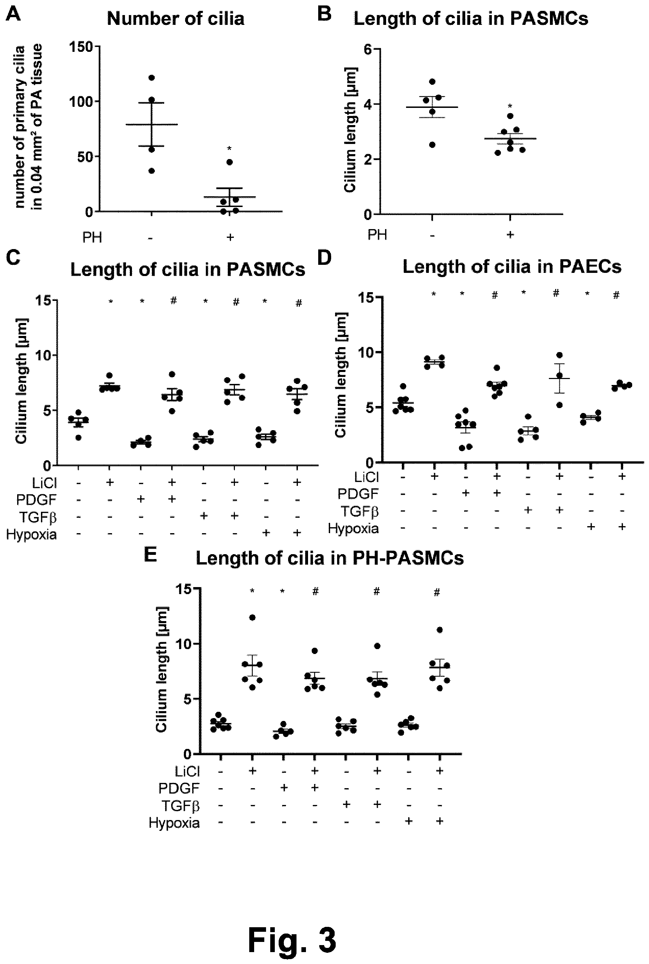

The primary cilium is an antenna-like organelle made by most human cells can be expressed and used as a mechano-sensor and signal node controlling important cellular processes such as cell proliferation. Fig. 3, Fig. 4, Fig. 5 and Fig. 6 show a functional correlation of cilia changes and pulmonary hypertension. Furthermore, the stabilizing effect of lithium on the cilia is demonstrated. Hence, by establishing a correlation between PH and cilia deterioration, Fig. 3, Fig. 4, Fig. 5 and Fig. 6 further support the suitability of Lithium in the treatment or prevention of pulmonary hypertension.

### Short description of the Figures

The following sections gives a summary for the Figures:
Fig. 1A shows the influence of Lithium on the proliferation in PASMCs.
Fig. 1B shows the influence of Lithium on the hypertrophy in PASMCs.
Fig. 1C shows the influence of Lithium on the migration in PASMCs.
Fig. 1D shows the influence of Lithium on PH under chronic hypoxia.
Fig. 2 shows a correlation between the occurrence of PH and diseases in which lithium therapy is used.
Fig. 3A: shows a reduction of the number of cilia in PASMC.
Fig. 3B shows a reduction of the length of cilia in PASMC.
Fig. 3C shows the effect of PH stimuli such (hypoxia, TGFβ, and PDGF, and Lithium salts) on shortening of the cilium in PASMC.
Fig. 3D shows the effect of PH stimuli such (hypoxia, TGFβ, and PDGF, and Lithium salts) on shortening of the cilium in PAEC.
Fig. 3E shows the effect of PH stimuli such (hypoxia, TGFβ, and PDGF, and Lithium salts) on shortening of the cilium in PASMC of PH patients.
Fig. 4 shows a correlation between the occurrence of PAH and ciliopathies.
Fig. 5 shows the absence of the endothelial primary cilium leading to a deterioration in PH parameters.
Fig. 6A, 6B shows the knock-down of IFT88 leading to accelerated cell migration, while rapamycin reduces it again.

### Detailed description of the Figures

The following section describes the Figures in more detail:
In Fig. 1, it is demonstrated how Lithium counteracts the PH phenotype. Lithium reduces proliferation (see Fig. 1A), hypertrophy (see Fig. 1B) and migration (see Fig. 1C) in PASMCs from healthy volunteers and in PASMCs from PH patients. Cells were incubated at ± 50 mM LiCI. In addition, food with 0.2% Li2CO3 prevents the development of a PH under chronic hypoxia (10% O₂ for 5 weeks) in mice (see Fig. 1D). Data are mean ± SEM. * p <0.05 vs. control; #p <0.05 vs. condition without lithium; n ~ 3.
In Fig. 2, a correlation analysis was carried out. The incidence of PH disease in patients with conditions treated with lithium was compared to the hypothetical incidence of the two conditions in the same patient if they were to occur independently. The frequency of PH is reduced in patients with diseases that are often treated with lithium.
In Fig. 3, demonstrates the effect of PH, PH stimuli and Lithium salts on cilia condition and development. The number (see Fig. 3A) and length (see Fig. 3B) of the primary chambers of pulmonary artery muscle cells (PASMC) are reduced in PH patients when compared to the PASMCs of healthy controls. Furthermore, classic PH stimuli such as hypoxia, TGFβ, and PDGF lead to a shortening of the cilium in PASMC (see Fig. 3C), pulmonary arterial endothelial cells (PAEC) but not in PASMC of PH patients. Lithium chloride leads to a significant lengthening of the cilia in all conditions (see Fig. 3C, Fig. 3D, and Fig. 3E). The cells were incubated in normoxia or hypoxia (1%O₂) ± 100 ng / ml PDGF, ± 5 ng / ml TGFβ and ± 5O mM LiCI. Data are mean ± SEM. * p<0.05 vs. control; #p <0.05 vs. condition without lithium; n ~ 3.

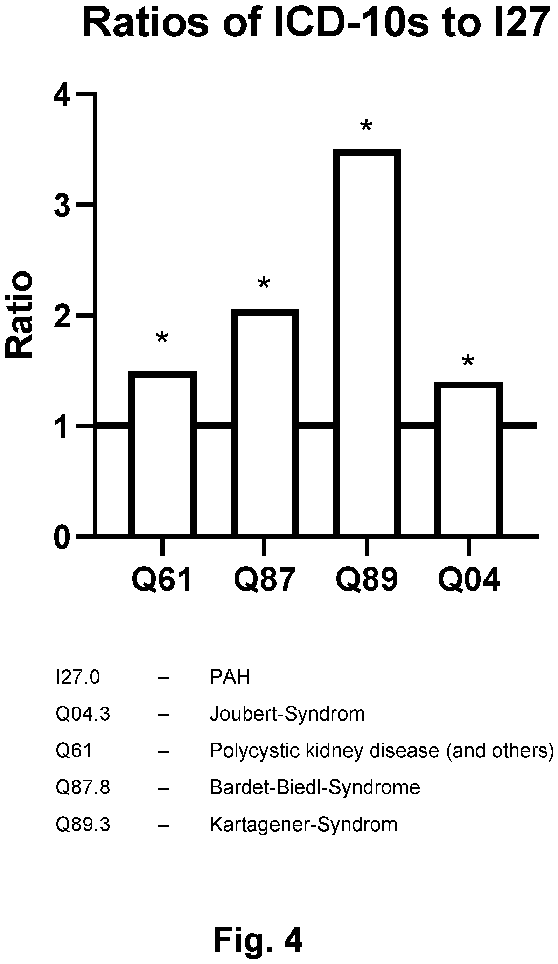

In Fig. 4, a correlation analysis was carried out. The incidence of PH disease in patients with ciliopathies was compared to the hypothetical incidence of the two conditions in the same patient if they were to occur independently. The frequency of PH in patients with ciliopathies is increased, which supports the assumption of a causal relationship between cilium defect and pulmonary hypertension development. Indirectly, these further findings supporting the presence of a causal relationship between cilium defect and pulmonary hypertension development serve as a clinically relevant indicator in that Lithium salts can be successfully used in the treatment of pulmonary hypertension. These results match the findings of the reduced frequency of PH in patients with diseases that are often treated with lithium (see also Fig. 2 and the corresponding text above).

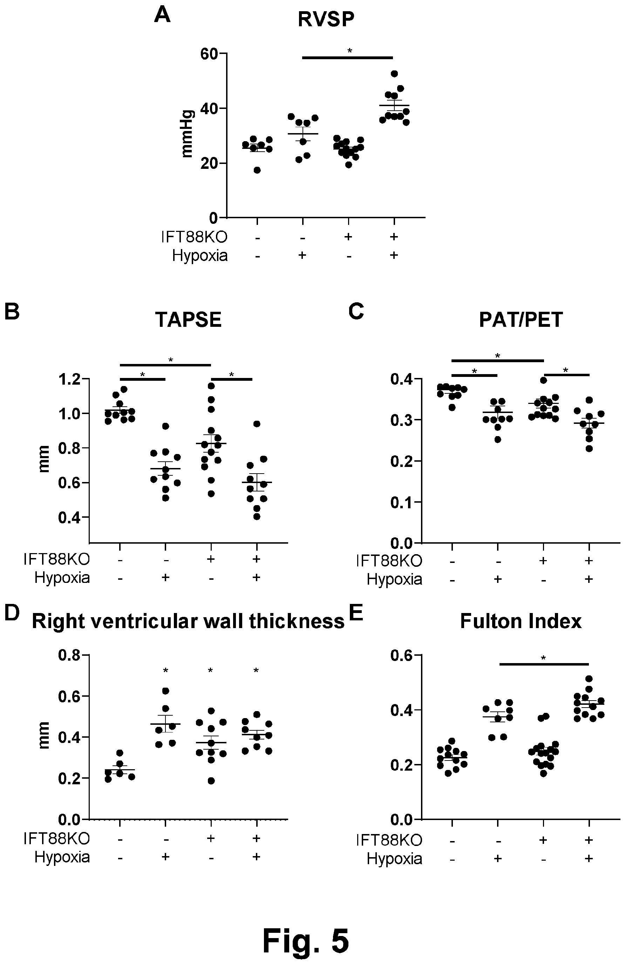

Fig. 5 shows the absence of the endothelial primary cilium leading to a deterioration in PH parameters. By using an endothelial-specific knock-out of IFT88, an essential protein for the ciliogenesis, it was possible to investigate the effect that the lack of primary cilia in endothelial cells exerts on the development of pulmonary hypertension in vivo. For this purpose, the mice were kept in hypoxia (10% O₂) for five weeks in order to induce pulmonary hypertension, or, as control animals, in normoxia. It is noticeable that the two echocardiographic parameters PAT / PET (pulmonary artery acceleration time / pulmonary artery ejection time) and TAPSE (tricuspid annular plane systolic excursion), both of which are used to determine pulmonary hypertension, are already reduced in IFT88-KO mice in normoxia compared to the control mice (Figure 5A, and Fig. 5B). Even if the difference between either hypoxia group is not significant, there is a tendency towards a further deterioration of the two parameters in the IFT88-KO mice. With regard to right ventricular hypertrophy, measured using the right ventricular wall thickness (Figure 5C) and the Fulton index (Figure 5D), there is a deterioration in the parameters between knockout mice and the controls in normoxia (C) or hypoxia (D). visible. The Fulton index is the weight of the right ventricle compared to the weight of the left ventricle and septum. A change of 30% between IFT88-KO mice and the corresponding control animals after 5 weeks of hypoxia can also be seen in the right ventricular systolic blood pressure (RVSP, Figure 5E). The data all suggest that the absence of the cilium favors the development of pulmonary hypertension. The data further supports the finding that Lithium salts can be successfully used in the treatment of pulmonary hypertension. Indirectly, these further findings supporting the presence of a causal relationship between cilium defect and pulmonary hypertension development serve as a clinically relevant indicator in that Lithium salts can be successfully used in the treatment of pulmonary hypertension.

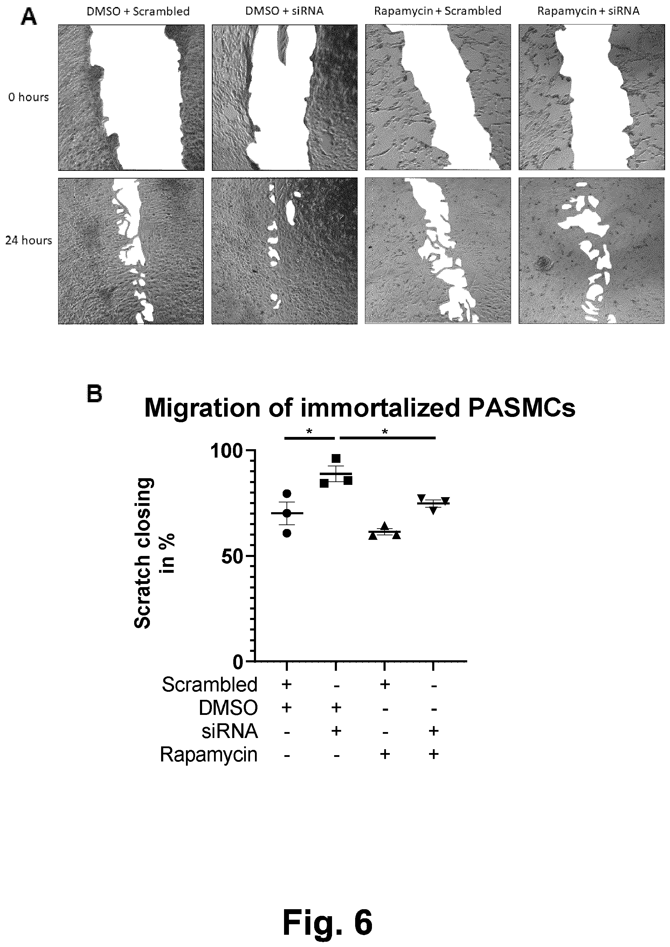

Fig. 6 shows the knock-down of IFT88 leading to accelerated cell migration, while rapamycin reduces it again. The connection between cilium deregulation and pulmonary hypertension development could be mediated via the mTOR signaling pathway (mammalian target of rapamycin). In order to assess this assumption, an IFT88 knock-down was carried out using siRNA in vitro and analyzed the migration behavior of immortalized pulmonary arterial vascular muscle cells. As previously stated, the knock-down of IFT88 led to accelerated cell migration. Rapamycin, an inhibitor of the mTOR signaling pathway, was able to normalize this behavior again (see Figure 6A: photos; Figure 6B: data plot). The inhibition of the mTOR signal path thus eliminates the in the behavior of cells as caused by the loss of cilia. These findings indicate that cilia loss via activation of mTOR leads to increased migration of vascular muscle cells in the case of pulmonary hypertension. Indirectly, these further findings supporting the presence of a causal relationship between cilium defect and pulmonary hypertension development serve as a clinically relevant indicator in that Lithium salts can be successfully used in the treatment of pulmonary hypertension.

### Summary

The experimental data demonstrate the effect of Lithium on pulmonary hypertension, thereby illustrating the use of lithium salts in the treatment or prevention of pulmonary hypertension. In particular, the protective effects of lithium on typical cellular reactions of pulmonary hypertension patients could be shown in proof-of-principle experiments, confirmed in animal experiments. The administration of lithium carbonate reduced the increase in the right ventricular systolic blood pressure as the primary endpoint in preclinical investigations for pulmonary hypertension

Furthermore, a functional correlation of cilia changes and pulmonary hypertension is established. Furthermore, in the experiments carried out in the context of the present invention, lithium was able to elongate the primary cilium significantly. Given the stabilizing effect of lithium on cilia, establishing the correlation between PH and cilia deterioration provides further support for the suitability of lithium in the treatment or prevention of pulmonary hypertension.

### Methods:

Ration analysis: In order to calculate the probability of different diseases developing in the same patient, a database of all patients admitted to the Charité and their corresponding ICD-10 values was analyzed. If the same patient is admitted to the hospital at another time point, both diseases are recorded for the same patient. Using this database, we were able to calculate the ratios between diseases by dividing the expected values and observed values of two diseases appearing in the same patient. The expected value is the basic assumption that both diseases develop independently of each other; the observed value is the actual probability of both diseases appearing in the same patient.

Cilium count in human tissue: Ten µm cryoslides of pulmonary arteries of patients with different types of PH and of healthy donors were washed with PBS and fixed in 4% formaldehyde. Fixed tissue was treated with 50 mM ammonium chloride, washed, permeabilized with 0.3% Triton-X in PBS, washed, blocked in 3% BSA for one hour and stained for acetylated α-tubulin and smooth muscle actin (both 1:400 in 3%BSA/PBS) overnight at 4°C. Next day, tissue was washed, treated with secondary antibody (1:500 in 3% BSA/PBS), washed, treated with DAPI, washed and mounted on microscope slides. Samples were analyzed by imaging of z-stacks using confocal microscopy. The number of cilia was counted with Imaris analysis software.

General cell culture: Experiments were done with cells in passage 4-7. Pulmonary arterial smooth muscle cells (PASMCs) from PH patients or healthy donors were cultured in smooth muscle cell growth medium and pulmonary arterial endothelial cells (PAECs) were cultured in endothelial cell growth medium. Cells were grown until 70-90% confluence, then washed with PBS and detached with accutase.

Proliferation assay: 10.000 cells were seeded out in 96-well plates in 200 µl medium with or without 50 mM lithium chloride. 20 µl BrdU was added according to the BrdU cell proliferation kit protocoll. 24 hours later, cells were washed and fixed and further treated following the protocol.

Hypertrophy assay: 100.000 cells were seeded out in 12-well plates in 1 ml medium. After reaching 70-90% confluency, medium was changed and 50 mM lithium chloride was or was not added. After 24 hours, cells were washed twice with PBS. 20 µl ice cold RIPA buffer were added, the cells were scraped and transferred into a tube. The cells were frozen three times with liquid N2 and were thawed each time on ice. Cells were centrifuged 15' at full speed and the supernatant was transferred to a new tube. Protein concentration was measured. 20 µg protein were loaded into a 10% SDS gel and run for ca. 60 minutes at 90-120 V. The gel was wet-blotted onto a nitrocellulose membrane at 90 V for 90'. The membrane was blocked with 5% BSA in TBS-T for 1 hour and then incubated in 1:1000 anti-SMA in 5% BSA/TBS-T over night. The membrane was washed three times in TBS-T, incubated in 1:10000 a-mouse antibody and washed again. ECL solution was applied and the membrane was imaged. Afterwards, the membrane was washed, stripped and incubated with 1:1000 anti-GAPDH for 1 hour. Secondary antibody treatment was the same as before. The band intensity was measured with ImageJ and hypertrophy was assessed by dividing the SMA-band by the GAPDH-band.

Migration assay: 100.000 cells were seeded out in 12-well plates in 1 ml medium. After reaching 70-90% confluency, medium was changed and 50 mM lithium chloride was or was not added. Additionally, a scratch was made with a pipette tip. A picture was taken. After 24 hours, a picture was taken again. The closing of the scratch was determined with ImageJ by comparing both pictures.

Cilium measurements: 100.000 cells were seeded out on gelatin-coated coverslips in 12-well plates. After reaching confluency, ciliogenesis was stimulated by exchanging the medium to serum-free medium for 48 hours. Afterwards, medium was changed to serum-free medium containing or not containing 50 mM LiCI and 5 ng/ml TGFβ or 100 ng/ml PDGF or the cells were put into hypoxia (1% O2). After 24 hours, cells were washed with PBS and fixed in 4% formaldehyde. Fixed cells were treated with 50 mM ammonium chloride, washed, permeabilized with 0.3% Triton-X in PBS, washed, blocked in 3% BSA for one hour and stained for Arl13b and acetylated α-tubulin (both 1:400 in 3%BSA/PBS) overnight at 4°C. Next day, cells were washed, treated with secondary antibody (1:500 in 3% BSA/PBS), washed, treated with DAPI, washed and mounted on microscope slides. Cilia were visualized with confocal microscopy and length was measured with ImageJ.

IFT88 knock-down migration assay: 100.000 cells were seeded out in 12-well plates in 1 ml medium. 24 hours later cells were washed with serum-free medium and siRNA against IFT88 (or scrambled siRNA for the control) in a final concentration of 46,1 nM was added according to the effectene kit protocol. After 4 hours, 1 ml of medium was added. After 24 hours, medium was changed. After 48 hours, conditioned medium -- rapamycin in DMSO (final concentration 200 nM) or DMSO only - was added. Additionally, a scratch was made with a pipette tip. A picture was taken. After 24 hours, a picture was taken again. The closing of the scratch was determined with ImageJ by comparing both pictures.

Animal experiments:
General: 10-12 week old male C57BL/6-mice were kept at normoxia or in a hypoxia chamber at 10% O2 for five weeks and got either regular chow or chow containing 0.2% Li2CO3.
IFT88 knock-out in mice: IFT88-flox mice were bred with Cdh5-cre mice. 2 weeks prior to treatment, IFT88fl/fl, Cdh5-cre+ and Cdh-cre- mice were intraperitoneally injected with tamoxifen (75 mg/kg body weight) to induce knock-out.
Echocardiography: Mice were anesthetized with initially 3% isoflurane. The animals are fixed in dorsal position and their thoracal hair was removed with depilatory cream. Pulmonary artery flow, tricuspid valve movement and other echocardiographic parameters were assessed with the echo device Vevo3100.
RVSP measurement: After five weeks, the mice got an intraperitoneal injection of ketamin (200 mg/kg bodyweight (bw)) and xylazine (10 mg/kg bw). A 2F microtip millar catheter was advanced into the right ventricle via the jugularis vein to measure right ventricle systolic pressure (RVSP).

**Materials:**

| | |
|---|---|
| Smooth Muscle Cell Growth Medium 2 | Promocell (C-22062) |
| Endothelial Cell Growth Medium | Promocell (C-22010) |
| PAECs | Promocell (C-12241) |
| BrdU Cell Proliferation ELISA Kit | Abcam (ab126556) |
| a-SMA | Abcam (ab7817) |
| a-GAPDH | Abcam (ab8245) |
| a-acetylated α-tubulin | Cell signaling (5335) |
| a-Arl13b | Abcam (ab136648) |
| a-mouse Alexa Fluor 594 | Invitrogen (A11032) |
| a-rabbit Alexa Fluor 488 | Invitrogen (A11008) |
| a-mouse-HRP | GE Healthcare/Cytiva (NA931) |
| a-rabbit-HRP | Abcam (ab97051) |
| ON-TARGETplus siRNA IFT88 | Horizon (LQ-012281-01-0002) |
| Effectene Transfection Reagent | Qiagen (301425) |

## Claims

1. A lithium salt for use in treatment or prevention of pulmonary hypertension

2. The lithium salt according to claim 1, wherein the pulmonary hypertension is pulmonary arterial hypertension (PAH, WHO group I).

3. The lithium salt according to claims 1 or 2, wherein the pharmaceutically acceptable lithium salt is selected from lithium chloride, lithium carbonate, lithium sulfate and lithium citrate.

4. The lithium salt for use according to anyone of claims 1 or 3, wherein the lithium salt is for use by topical or systemic administration, preferably intravascular, intravenous, intra-arterial, intracardial, intra-pulmonal and/or nasal administration; in particular oral administration.

5. A pharmaceutical composition for use in treatment or prevention of pulmonary hypertension, wherein the pharmaceutical composition comprises the pharmaceutically acceptable lithium salt according to one of the preceding claims and a pharmaceutically acceptable excipient.

6. The pharmaceutical composition for use according to claim 5, wherein the pulmonary hypertension is pulmonary arterial hypertension (PAH, WHO group I).

7. The pharmaceutical composition for use according to anyone of claims 5 or 6, wherein the pharmaceutical composition is configured for topical or systemic administration, preferably for intravascular, intravenous, intra-arterial, intracardial, intra-pulmonal and/or nasal administration, in particular oral administration.
